# EUROPEAN PATENT SPECIFICATION

(11) **EP 4 453 526 B1**
(45) Date of publication and mention of the grant of the patent: **01.04.2026**
(21) Application number: 22839792.3
(22) Date of filing: 19.12.2022
(51) Int. Cl.: G01L 9/00, G01L 19/00, G01L 19/06, G01L 19/08, G01L 19/14, A61B 5/00

(54) **PRESSURE SENSOR**
DRUCKSENSOR
CAPTEUR DE PRESSION

(30) Priority: 21.12.2021 EP 21216355
(43) Date of publication of application: 30.10.2024
(73) Proprietor: BIOTRONIK SE & Co. KG, 12359 Berlin (DE)
(72) Inventor: SKERL, Olaf, 18209 Bad Doberan (DE); VAN OOYEN, André, 10717 Berlin (DE); ROMBERG, Jan, 12347 Berlin (DE)
(74) Representative: Biotronik Corporate Services SE
(86) International application number: PCT/EP2022/086579
(87) International publication number: WO 2023/117856

(56) References cited:
- EP-A2- 0 145 146
- US-A1- 2013 247 644

## Description

The present invention relates to a pressure sensor for pressure measurement in an aggressive medium.

Pressure sensors for use in a medium generally include a movable element, such as a membrane. It must be adapted for determining a pressure difference between an inner side of the movable element and an outer side of the movable element which stays in contact with the medium of interest. As this concept has seen continued research and development, issues regarding precise pressure measurements have come to light. Since in sensors comprising a membrane the pressure is generally determined based on a bending of the membrane caused by the surrounding medium, particularly the information transfer of the degree of bending to an electrical evaluation circuit and/or a transducer is critical.

There have been attempts in the prior art to arrange the evaluating circuit/transducer inside a cavity filled with a coupling liquid. In this manner, the membrane basically prevents a mixing of the coupling liquid and the surrounding medium, while the external pressure applied to the membrane is directly transferred over the coupling liquid onto a transducer inside the cavity. However, thermal expansion and aging effects of the coupling liquid negatively influence precise pressure measurements. Furthermore, already small bubbles inside the coupling liquid introduced during a filling process incorporate measurement errors, which are not easily compensated by any evaluation routine.

Alternatively, instead of using a coupling liquid inside the pressure sensor, attempts have been made, for example in prior art document US 2005/0160823, to attach strain transducers, such as piezoelectric or piezoresistive materials or similar, directly onto the membrane to receive information about a degree of membrane bending which can be utilized for determining the external pressure. However, creeping of the fixing material or adhesive required for attaching the strain transducer on the membrane mostly leads to a high drift over time for such pressure sensors. Furthermore, attaching a plurality of strain transducers on the membrane not only requires a complex and costly manufacturing process, but also massively influences the behavior of the membrane, leading to additional uncertainties for the pressure value of interest.

A further alternative known in the art is to determine the pressure based on the functional principal of a capacitor in which the membrane corresponds to one capacitor plate. In this manner, a pressure induced movement of the membrane leads to a change in an electric signal, e.g., a capacitance or a voltage, which can be used for deriving the external pressure. However, using the membrane as capacitor electrode increases the sensitivity for noise and additional measurement uncertainties, since the membrane and the evaluation circuitry are in electrical contact. Furthermore, since the membrane is generally in contact with the medium surrounding the sensor, any fluctuations of the (electric) potential of the medium directly influences the determined pressure value negatively.

Moreover, further problems arise when utilizing known pressure sensors in an aggressive medium, which reacts with the sensor's material and thus leads to deterioration of the membrane or a housing of the sensor, for example. Additionally, piezoresistive materials or structures are generally based on silicon with an outer passivation layer based on silicon oxide or silicon nitride. However, both the inner silicon layers as well as the outer oxide layers are generally not stable in most aggressive media. This may result in malfunctioning pressure sensors or unreliable pressure values.

An implantable pressure sensor within a living being is known from US 2013/247644 A1.

It may therefore be considered as a problem underlying the present invention to provide an improved pressure sensor for pressure measurement in an aggressive medium that avoids at least in part the difficulties encountered in the prior art.

The above problem may at least partly be solved by a pressure sensor as defined in claim 1. Such a pressure sensor may allow for a more precise and temporally stable pressure measurement in an aggressive medium so that the above outlined disadvantages of the prior art may at least partly be overcome.

By providing a pressure sensor with a flexible element adapted for determining the external pressure and a separate movable element, which may be in contact to the aggressive medium, the problems of the prior art can be addressed or resolved individually.

For example, a design and/or configuration of the flexible element can be optimized independently from a design and/or configuration of the movable element. Further, a material of the movable element may be particularly tailored for compatibility with the surroundings of the sensor, e.g. blood. Hence, materials that are particularly stable even in aggressive media may be used. On the other hand, the flexible element can be individually optimized for enabling a high-precision and improved pressure determination without a drawback of being limited to materials stable in the aggressive medium. By this, the material of the flexible element can be selected solely based on its sensing properties without requiring any degree of resistance against the aggressive medium, since there is no contact between the flexible element and the aggressive medium.

The pressure sensor is an implantable pressure sensor. The implantable pressure sensor is adapted for being inserted into a human or animal body and into a blood circulation for determining a blood pressure, for example in a blood vessel and/or heart. In this embodiment, the aggressive medium may be blood.

The base element and the movable element may at least in part form a hermetically sealed cavity. The cavity may comprise a known reference pressure, preferably vacuum. The flexible element may be arranged within the hermetically sealed cavity. By this, the flexible element may be protected/shielded by the cavity from getting in contact with the aggressive medium and/or noise generating electric stray fields. The movable element and the base element may be attached to each other by welding, gluing, bonding, soldering or similar methods known in the art, wherein welding may be preferred.

The movable element may comprise a membrane and/or a tappet arranged on a membrane. An individual manufacture of the membrane and the tappet may be provided. Further, utilizing different materials for manufacturing the membrane and the tappet may be applicable. Thus, each material may be specifically selected and/or optimized for its intended application. Alternatively, membrane and tappet may be manufactured integrally (e.g. monolithically), saving an additional and/or elaborate attachment step of the tappet onto the membrane. Further, an accuracy of the movable element may be improved by embodying the membrane and/or the tappet integrally.

A surface of the movable element facing towards the flexible element comprises a protrusion, preferably a tapered protrusion. The protrusion is in mechanical contact with the flexible element. In this manner, a contacting area of the movable element and the flexible element may be reduced. Thus, a precise and/or essentially homogeneous contacting area can be provided, which may improve a pressure measurement accuracy. On the contrary, when utilizing a large contacting area, already a small misalignment of the movable element and the flexible element, e.g. based on a small deviation from a parallel arrangement, can lead to an inhomogeneous contacting area, which negatively influences the pressure measurement. Small misalignments can be caused by manufacturing uncertainties and/or an inhomogeneous external pressure applied by the aggressive medium across the movable element, for example. Thus, a small contacting area, e.g. below 10 %, preferably below 5 % and most preferably below 2 % of an extent of the flexible element perpendicular to a bending/flexion direction may be preferred (the extent being, for example the unsupported length of a doubly clamped beam or the length of a singly clamped beam, etc.).

The protrusion exerts a mechanical force on the flexible element.

The mechanical force depends at least in part on a position of the movable element. In this manner, a force applied by the aggressive medium onto the movable element can be transferred onto the flexible element. Since the position of the movable element depends at least in part on a magnitude of the external pressure, the magnitude of the mechanical force is directly based on the external pressure. For example, for an external pressure p1 > p2, a distance of the movable element to the base element may be smaller for p1 than for p2. Furthermore, a smaller contacting area of the movable element and the flexible element may generate a more homogenous mechanical force onto the flexible element. Thereby, a reliability and/or an accuracy of the pressure sensor may be improved.

The flexible element may comprise at least one piezoresistive material or structure. As generally known, a mechanical force applied onto the piezoresistive material or structure can generate a change of the electrical resistance across the piezoresistive material or structure. The magnitude of the resistance change thereby depends on the magnitude of the mechanical force. The applied mechanical force can thus be determined by directly or indirectly measuring the generated electrical resistance change or by determining any other electric signal which depends on the mechanical force. In this manner, the flexible element comprising at least one piezoresistive material or structure may be adapted for determining a strain and/or flexion of the flexible element based on the piezoresistive material or structure. The strain may be caused at least in part by a mechanical force applied by the movable element. Since the movable element may be moved based at least in part on an external pressure of the aggressive medium, the strain and/or flexion may depend at least in part on the external pressure of the aggressive medium. Thus, by determining the generated electric resistance change (directly or indirectly) the external pressure can be determined. Additionally or alternatively, any means for measuring a curvature of the membrane in contact with the aggressive medium can be avoided. As known in the prior art, such means generally influence the behavior of the membrane negatively resulting in an unreliable and/or imprecise pressure measurement. Moreover, no electric connection of said membrane nor any defined electric potential of said membrane may be required. This may allow for selecting the membrane's material and/or its design solely based on and optimized for the aggressive medium of its intended application. The piezoresistive material or structure may for example be provided as a piezoresistive circuit on the flexible element. Alternatively, for example, the flexible element may essentially consist of a piezoresistive material.

The flexible element may comprise more than one piezoresistive circuits. The term "piezoresistive circuit" as used in the present invention may be understood as an electrical circuit including a piezoresistive material or structure, which circuit may be adapted for determining any generated electric resistance change (directly or indirectly) across the piezoresistive material or structure based on a flexion/bending of the flexible element. By arranging multiple piezoresistive circuits across the flexible element, a more precise pressure measurement can be provided. For example, if a pair of piezoresistive circuits would be arranged on opposite sides of the flexible element in bending direction, the piezoresistive material or structure of one circuit would be squeezed and the other one would be stretched while applying a force onto the flexible element in bending direction. Thus, a sign of the generated electric resistance change would be different. In this example, an identical sign of both generated electric resistance changes would indicate a squeezing/stretching unrelated to an applied force, e.g., based on thermal expansion of the flexible element or other reasons. Therefore, an arrangement of multiple piezoresistive circuits across the flexible element may improve an accuracy of the pressure measurement. Additionally or alternatively, utilizing multiple piezoresistive circuits can provide a functioning pressure sensor even with one or even some of the piezoresistive circuits malfunctioning. Thus, redundant information can be provided.

In some embodiments, the more than one piezoresistive circuits may be electrically connected as resistor bridge. As generally known in the art, a resistor bridge can be applied for determining a difference between multiple resistors instead of the actual quantity of a resistor directly. By this, it is possible to determine even small resistance differences even for large resistors, which otherwise, i.e. via direct resistance measurement, would be difficult to resolve. In this manner, by connecting multiple piezoresistive circuits in a resistor bridge arrangement, even small generated electric potentials and/or small degrees of bending/flexing of the flexible element can be precisely determined. A typical example of a resistor bridge arrangement is known in the art as Wheatstone bridge, for example.

The flexible element may comprise a one-sided clamped beam, a two-sided clamped beam, a circular membrane or a rectangular membrane. Depending on its intended application, selecting one of the above options provides a very versatile pressure sensor. For example, a bending/flexion of a one-sided clamped beam may be larger and thus more reliable in determining small pressure values compared to a two-sided clamped beam or a membrane, if applying identical forces, for example. However, a two-sided clamped beam and/or a membrane may be less prone to errors based on external influences, e.g. vibrations of the pressure sensor. Further, a membrane may enable to arrange more piezoresistive circuits symmetrically around a contacting area of the movable element and the flexible element (compared to e.g., a one-sided or two-sided clamped beam), thus enabling a more precise pressure measurement. Thus, selecting an optimized shape and/or design of the flexible element based on its intended application can be provided.

The flexible element may be manufactured integrally. By this, an additional and/or elaborate attachment step of the at least one piezoresistive material or structure on the flexible element can be avoided. Further, an accuracy of the flexible element and/or a precise arrangement of the at least one piezoresistive material or structure may be improved by integrally manufacturing the flexible element. In some embodiments, the flexible element may be manufactured using a microelectromechanical system, MEMS, technology (or a nanoelectromechanical, NEMS, technology). In this manner, a cost efficient and highly precise/reproducible integrally manufacturing technique may be provided. For example, the piezoresistive material or structure may be based on silicon, which is known in the art as a material enabled for manufacturing of MEMS. Further, simultaneous manufacture of multiple flexible elements on a wafer may be provided. Alternatively, a piezoresistive material or structure may be attached to the flexible element.

The movable element may comprise a first rigidity and the flexible element may comprise a second rigidity. Based thereon, a characteristic of the pressure sensor may be essentially determined by the one of the flexible element or the movable element, having a higher rigidity. Thereby a complexity of an evaluation algorithm for determining a pressure of the aggressive medium may be reduced, which increases an efficiency of the pressure sensor according to the present invention. This may be particularly relevant for an implantable pressure sensor, which comprises limited energy resources.

A height of the pressure sensor may be between 0.5 and 5.0 mm, preferably between 1.5 and 4.0 mm, and more preferably 3 mm. A length of the pressure sensor may be between 1 and 10 mm, preferably between 2 and 7 mm, and more preferably 3 mm. In this manner, the pressure sensor may be suitable to be implanted into a human or animal body to, for example, measure a blood pressure.

The base element may comprise or may essentially consist of titanium, stainless steel, glass, ceramic, or glass-ceramic. For example, the base element may be arranged on an additional part of a device for measuring a pressure, for example a housing of the device (e.g., including a battery, a wireless transmitter or transceiver, etc.), or arranged next to an additional (similar or different) sensor. In this manner, the material of the base element may be adapted to the material of the additional part or sensor. The movable element may comprise or may essentially consist of titanium, stainless steel, glass, ceramic, or glass-ceramic, preferably titanium. The movable element and the base element may comprise the same material. Alternatively, the movable element and the base element may comprise different, e.g. individually optimized materials. The potential of utilizing various materials for the base element and/or the movable element may enable to specifically select each material based on an intended surrounding of the pressure sensor, e.g. blood.

In some embodiments, all surfaces of the movable element and/or the base element, which are in direct contact to the aggressive medium, may be biocompatible and/or bio-stable. In this manner, a degradation of the pressure sensor over time due to its surroundings, e.g. blood, can be avoided, or at least reduced. Further, an unintended immune response or reaction of a patient's body, in which the pressure sensor according to the present invention may have been implanted, can be prevented, or at least reduced by a great extent.

The pressure sensor may further comprise means for determining an electrical signal caused by a strain in the flexible element based at least in part on a movement of the movable element. The means for determining the electrical signal may be included in an applicationspecific integrated circuit, ASIC. Generally, an ASIC corresponds to a small integrated circuit customized for a particular use, rather than intended for general-purpose use. ASICs in mass production provide a cost efficient and reliable integrated circuit. Further, the flexible element may be arranged on the ASIC and/or arranged next to the ASIC. In this manner, a size of the sensor may be reduced. The ASIC may be in electrical contact to the at least one piezoresistive material and may be adapted to determine a strain of the flexible element.

The pressure sensor may further comprise means to wirelessly transmit the determined electrical signal or any information based on the determined electrical signal (e.g. as a raw signal or a calibrated pressure signal, etc.) as analogue or digital signal. This may be particularly relevant if the pressure sensor cannot be directly wired based on its location, for example when being implanted into a human or animal body. The pressure sensor may further comprise or may be connected to powering means, e.g. a battery.

Aspects of the present invention are described in more detail in the following by reference to the accompanying Figure. The Figure shows:

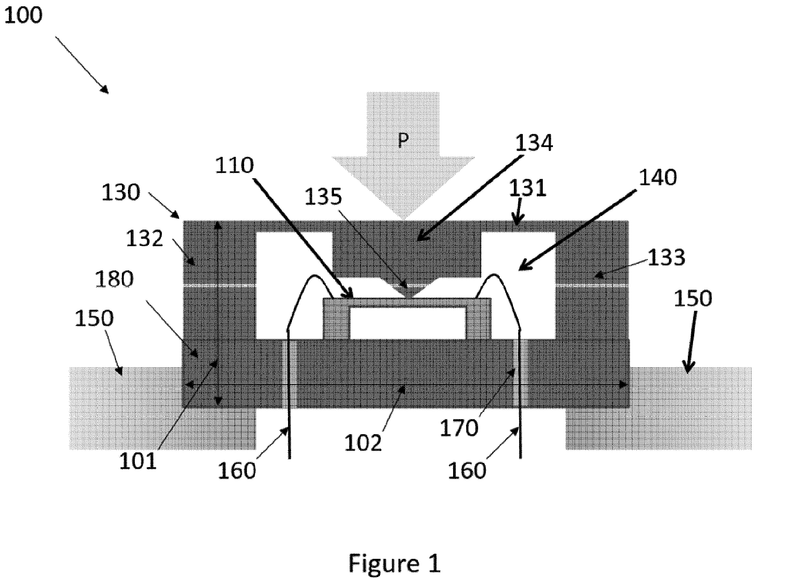

- Fig. 1: an illustration of a cross sectional view of an embodiment of a pressure sensor according to the present invention.

In the following, exemplary embodiments of the present invention are described in more detail, with reference to a pressure sensor. While specific feature combinations are described in the following with respect to the exemplary embodiments of the present invention, it is to be understood that the disclosure is not limited to such embodiments. In particular, not all features have to be present for realizing the invention, and the embodiments may be modified by combining certain features of one embodiment with one or more features of another embodiment.

Fig. 1 depicts a cross section of an embodiment of a pressure sensor 100 for pressure measurement in an aggressive medium according to the present invention. The pressure sensor 100 comprises a flexible element 110 configured as a two-sided clamped beam adapted for determining an external pressure. Other configurations of the flexible element 110, e.g. as a one-sided clamped beam, a circular membrane or a rectangular membrane are also applicable. The flexible element 110 is arranged on a base element 180, e.g. a base plate. The base element 180 may comprise or may essentially consist of titanium, stainless steel, glass, ceramic, or glass-ceramic, wherein titanium is generally preferred. The flexible element 110 may be a silicon-MEMS including at least one piezoresistive material or structure, e.g. a piezo resistor. The piezoresistive material or structure is adapted for and/or used for determining a strain of the flexible element 110. In a preferred embodiment, various piezoresistive circuits/piezo resistors are included in the flexible element 110 and electrically connected in a resistor bridge arrangement. In this embodiment, conductive wires 160 are connecting the resistor bridge to means for determining an electrical signal caused by a strain in the flexible element (not shown). The determined signal may be a detuning signal of the resistor bridge indicating a flexion/bending of the flexible element 110. When the conductive wires 160 are guided through a conductive element, e.g. base element 180, an insulating feed-through 170, e.g. made from ceramics, can be utilized for insulating the conductive wire 160 from the conductive element of the pressure sensor 100. The insulating feed-through may be pressure tight.

The pressure sensor 100 further comprises a movable element 130, which is adapted to move based at least in part on an external pressure (change). The movable element 130 is integrally formed and includes a membrane 131, a left sidewall 132, a right sidewall 133, a tappet 134 and a tapered protrusion 135. The movable element 130 may comprise or may essentially consist of titanium, stainless steel, glass, ceramic, or glass-ceramic, preferably titanium. An individual manufacturing process combined with an additional attachment process of some or even all parts 131-135 of the movable element 130 may also be applicable.

The movable element 130 together with the base plate 180 may form a cavity 140 enclosing the flexible element 110. Thereby the flexible element 110 does not get in contact with the aggressive medium surrounding the pressure sensor 100. The cavity 140 may be hermetically sealed including a known reference pressure, preferably vacuum. Other pressure values, e.g., atmospheric pressure or excess pressure, may also be applicable and selected based on an intended application of pressure sensor 100.

The membrane 131 may be in contact with the aggressive medium. Further, the membrane 131 is configured as part of a moveable element 130 and to move at least in part based on an external pressure (change) caused by the aggressive medium. A thickness of the membrane 131 may preferably be selected from the range from 2 µm to 100 µm, 5 to 50 µm, or 15 µm to 25 µm, while other thicknesses are also applicable. As can be seen from Fig. 1, tappet 134, tapered protrusion 135 and membrane 131 are integrally formed such that the tapered protrusion 135 is in mechanical contact with the flexible element 110. In this manner, a movement of the membrane caused by the external pressure (change) simultaneously moves the tappet 134 and the tapered protrusion 135 closer to/further away from the base element 180 for increasing/decreasing external pressure, thereby applying a mechanical force onto the flexible element 110.

The pressure sensor 100 may further comprise means (not shown) for determining an electrical signal caused by a strain in the flexible element 110 based at least in part on a movement of the movable element 130. The strain may depend on the mechanical force applied by the movable element 130 on the flexible element 110. The electrical signal can correspond to an electric resistance change generated by a piezoresistive material or structure included in the flexible element 110, for example. In some embodiments, various piezoresistive circuits are arranged/included in the flexible element 110 in a resistor bridge arrangement and the means for determining may be adapted for determining a detuning signal of the resistor bridge arrangement.

The pressure sensor 100 may further comprise a transmitting unit and an antenna (not shown) for wirelessly transmitting the determined strain and/or flexion of the flexible element 110 as electrical signal, or any information (e.g., the detuning signal of the resistor bridge) based on the determined strain as analogue or digital signal. The pressure sensor 100 may further comprise a receiving unit (not shown), which is adapted to receive wirelessly transmitted signals. Receiver and transmitter may form a transceiver. The signals may include information required by the pressure sensor 100, such as calibration information or similar.

A height 101 of the pressure sensor 100 may be between 0.5 and 5.0 mm, preferably 3 mm. A length 102 of the pressure sensor 100 may be between 1 and 10 mm, preferably 3 mm. In this manner, the pressure sensor may be suitable to be implanted into a human or animal body to, for example, measure a blood pressure. The pressure sensor may have a generally round or rectangular shape and/or may comprise rounded edges.

As can be seen from Fig. 1, pressure sensor 100 is arranged on a further plate 150. Plate 150 may correspond to a housing of the pressure sensor device including the pressure sensor 100. Further, in case the pressure sensor 100 does not include any powering means (e.g. battery) or further means (e.g. transceiver), these may be arranged inside the pressure sensor device and connected to the pressure sensor 100. The pressure sensor device may also comprise means for anchoring it to tissue of a blood vessel or a heart, for example.

Most or even all outer surfaces of pressure sensor 100 may comprise material, which is resistant against the aggressive medium the pressure sensor 100 is intended to be used in and/or surrounded with. In particular, if pressure sensor 100 is implanted into a human or animal body, the material of the outer surfaces of pressure sensor 100 may be bio-compatible and/or bio-stable.

It may be noted that pressure sensor 100 may be fully functioning without requiring a coupling liquid inside the cavity or strain transducers attached to the membrane. Hence, pressure sensor 100 may be free from coupling liquid and no strain transducers may be attached to the membrane. Further, membrane 131 may not be required to be in electrical contact with any internal or external defined electric potential. In particular, membrane 131 can be electrically floating, and it may not be in electrical contact with the flexible element 110 and/or any means for determining an electrical signal caused by a strain/flexion in the flexible element 110. Moreover, most, or even all parts of pressure sensor 100 may be manufactured using a well-established and known in the art micro-electromechanical system (MEMS) technology having low manufacturing uncertainties while being cost-efficient.

## Claims

1. An implantable pressure sensor (100) for pressure measurement in an aggressive medium adapted for being inserted into a human or animal blood circulation comprising:
a base element (180);
a movable element (130) adapted to move at least in part based on an external pressure;
a flexible element (110) arranged on the base element (180) inside the pressure sensor (100) and adapted for determining the external pressure;
wherein the movable element (130) is mechanically in contact with the flexible element (110) via a protrusion at a surface of the movable element (130) facing towards the flexible element (110) and
wherein the flexible element (110) comprises at least one piezoelectric or piezoresistive material or structure .

2. The pressure sensor (100) according to claim 1, wherein the flexible element (110) is arranged within a hermetically sealed cavity (140), the cavity (140) formed at least in part by the base element (180) and the movable element (130).

3. The pressure sensor (100) according to any one of the previous claims, wherein the movable element (130) comprises a membrane (131) and/or a tappet (134) arranged on a membrane (131).

4. The pressure sensor (100) according to any one of the previous claims, wherein a surface of the movable element (130) facing towards the flexible element (110) comprises a tapered protrusion.

5. The pressure sensor (100) according to the previous claim, wherein the protrusion (135) exerts a mechanical force on the flexible element (110), wherein the mechanical force depends at least in part on a position of the movable element (130).

6. The pressure sensor (100) according to any one of the previous claims, wherein the flexible element (110) comprises more than one piezoelectric or piezoresistive circuit and wherein the more than one piezoelectric or piezoresistive circuits are electrically connected as resistor bridge.

7. The pressure sensor (100) according to any one of the previous claims, wherein the flexible element (110) comprises a one-sided clamped beam, a two-sided clamped beam, a circular membrane or a rectangular membrane.

8. The pressure sensor (100) according to any one of the previous claims, wherein the flexible element (110) is manufactured integrally.

9. The pressure sensor (100) according to any one of the previous claims, wherein the movable element (130) comprises a first rigidity and the flexible element (110) comprises a second rigidity;
wherein a characteristic of the pressure sensor (100) is essentially determined by one of the flexible element (110) or the movable element (130), having a higher rigidity.

10. The pressure sensor (100) according to any one of the previous claims, wherein the base element (180) comprises or essentially consists of titanium, stainless steel, glass, ceramic, or glass-ceramic.

11. The pressure sensor (100) according to any one of the previous claims, wherein the movable element (130) comprises or essentially consists of titanium, stainless steel, glass, ceramic, or glass-ceramic.

12. The pressure sensor (100) according to any one of the previous claims, further comprising means for determining an electrical signal caused by a strain in the flexible element (110) based at least in part on a movement of the movable element (130).

13. The pressure sensor (100) according to the previous claim, further comprising means for wirelessly transmitting the determined electrical signal or any information based on the determined electrical signal as analogue or digital signal.

## Patentansprüche

1. Implantierbarer Drucksensor (100) zur Druckmessung in einem aggressiven Medium, der zum Einführen in einen menschlichen oder tierischen Blutkreislauf ausgelegt ist, umfassend:
ein Basiselement (180);
ein bewegliches Element (130), das dazu ausgelegt ist, sich wenigstens teilweise basierend auf einem äußeren Druck zu bewegen;
ein flexibles Element (110), das auf dem Basiselement (180) innerhalb des Drucksensors (100) angeordnet und dazu ausgelegt ist, den äußeren Druck zu bestimmen;
wobei das bewegliche Element (130) über einen Vorsprung an einer dem flexiblen Element (110) zugewandten Oberfläche des beweglichen Elements (130) mechanisch mit dem flexiblen Element (110) in Kontakt steht und
wobei das flexible Element (110) wenigstens ein piezoelektrisches oder piezoresistives Material oder eine solche Struktur umfasst.

2. Drucksensor (100) nach Anspruch 1, wobei das flexible Element (110) innerhalb eines hermetisch abgeschlossenen Hohlraums (140) angeordnet ist, wobei der Hohlraum (140) wenigstens teilweise durch das Basiselement (180) und das bewegliche Element (130) gebildet wird.

3. Drucksensor (100) nach einem der vorhergehenden Ansprüche, wobei das bewegliche Element (130) eine Membran (131) und/oder einen auf einer Membran (131) angeordneten Stößel (134) umfasst.

4. Drucksensor (100) nach einem der vorhergehenden Ansprüche, wobei eine dem flexiblen Element (110) zugewandte Oberfläche des beweglichen Elements (130) einen sich verjüngenden Vorsprung umfasst.

5. Drucksensor (100) nach dem vorhergehenden Anspruch, wobei der Vorsprung (135) eine mechanische Kraft auf das flexible Element (110) ausübt, wobei die mechanische Kraft wenigstens teilweise von einer Position des beweglichen Elements (130) abhängt.

6. Drucksensor (100) nach einem der vorhergehenden Ansprüche, wobei das flexible Element (110) mehr als eine piezoelektrische oder piezoresistive Schaltung umfasst und wobei die mehr als eine piezoelektrische oder piezoresistive Schaltung elektrisch als Widerstandsbrücke verbunden sind.

7. Drucksensor (100) nach einem der vorhergehenden Ansprüche, wobei das flexible Element (110) einen einseitig geklemmten Balken, einen beidseitig geklemmten Balken, eine kreisförmige Membran oder eine rechteckige Membran umfasst.

8. Drucksensor (100) nach einem der vorhergehenden Ansprüche, wobei das flexible Element (110) einstückig hergestellt ist.

9. Drucksensor (100) nach einem der vorhergehenden Ansprüche, wobei das bewegliche Element (130) eine erste Steifigkeit und das flexible Element (110) eine zweite Steifigkeit umfasst;
wobei eine Besonderheit des Drucksensors (100) im Wesentlichen dadurch bestimmt wird, dass entweder das flexible Element (110) oder das bewegliche Element (130) eine höhere Steifigkeit aufweist.

10. Drucksensor (100) nach einem der vorhergehenden Ansprüche, wobei das Basiselement (180) Titan, Edelstahl, Glas, Keramik oder Glaskeramik umfasst oder im Wesentlichen daraus besteht.

11. Drucksensor (100) nach einem der vorhergehenden Ansprüche, wobei das bewegliche Element (130) Titan, Edelstahl, Glas, Keramik oder Glaskeramik umfasst oder im Wesentlichen daraus besteht.

12. Drucksensor (100) nach einem der vorhergehenden Ansprüche, ferner umfassend Mittel zum Bestimmen eines elektrischen Signals, das durch eine Dehnung im flexiblen Element (110) verursacht wird, wenigstens teilweise basierend auf einer Bewegung des beweglichen Elements (130).

13. Drucksensor (100) nach dem vorhergehenden Anspruch, ferner umfassend Mittel zum drahtlosen Übertragen des bestimmten elektrischen Signals oder beliebiger Informationen basierend auf dem bestimmten elektrischen Signal als analoges oder digitales Signal.

## Revendications

1. Capteur de pression implantable (100) destiné à la mesure de pression dans un milieu agressif adapté pour être inséré dans une circulation sanguine humaine ou animale comprenant :
un élément de base(180) ;
un élément mobile (130) adapté pour se déplacer au moins en partie en se basant sur une pression externe ;
un élément flexible (110) agencé sur l'élément de base (180) à l'intérieur du capteur de pression (100) et adapté pour déterminer la pression externe ;
dans lequel l'élément mobile (130) est mécaniquement en contact avec l'élément flexible (110) par l'intermédiaire d'une saillie au niveau d'une surface de l'élément mobile (130) orientée vers l'élément flexible (110) et
dans lequel l'élément flexible (110) comprend au moins un matériau ou structure piézoélectrique ou piézorésistant.

2. Capteur de pression (100) selon la revendication 1, dans lequel l'élément flexible (110) est agencé au sein d'une cavité hermétiquement scellée (140), la cavité (140) étant formée au moins en partie par l'élément de base (180) et l'élément mobile (130).

3. Capteur de pression (100) selon l'une quelconque des revendications précédentes, dans lequel l'élément mobile (130) comprend une membrane (131) et/ou un poussoir (134) agencé sur une membrane (131).

4. Capteur de pression (100) selon l'une quelconque des revendications précédentes, dans lequel une surface de l'élément mobile (130) orientée vers l'élément flexible (110) comprend une saillie effilée.

5. Capteur de pression (100) selon la revendication précédente, dans lequel la saillie (135) exerce une force mécanique sur l'élément flexible (110), dans lequel la force mécanique dépend au moins en partie d'une position de l'élément mobile (130).

6. Capteur de pression (100) selon l'une quelconque des revendications précédentes, dans lequel l'élément flexible (110) comprend plus d'un circuit piézoélectrique ou piézorésistant et dans lequel les plus d'un circuits piézoélectriques ou piézorésistants sont connectés électriquement en tant que pont de résistances.

7. Capteur de pression (100) selon l'une quelconque des revendications précédentes, dans lequel l'élément flexible (110) comprend une poutre serrée sur un côté, une poutre serrée sur les deux côtés, une membrane circulaire ou une membrane rectangulaire.

8. Capteur de pression (100) selon l'une quelconque des revendications précédentes, dans lequel l'élément flexible (110) est fabriqué d'un seul tenant.

9. Capteur de pression (100) selon l'une quelconque des revendications précédentes, dans lequel l'élément mobile (130) comprend une première rigidité et l'élément flexible (110) comprend une seconde rigidité ; dans lequel une caractéristique du capteur de pression (100) est essentiellement déterminée par l'un parmi l'élément flexible (110) ou l'élément mobile (130), ayant une rigidité supérieure.

10. Capteur de pression (100) selon l'une quelconque des revendications précédentes, dans lequel l'élément de base (180) comprend ou est essentiellement constitué de titane, d'acier inoxydable, de verre, de céramique, ou de vitrocéramique.

11. Capteur de pression (100) selon l'une quelconque des revendications précédentes, dans lequel l'élément mobile (130) comprend ou est essentiellement constitué de titane, d'acier inoxydable, de verre, de céramique, ou de vitrocéramique.

12. Capteur de pression (100) selon l'une quelconque des revendications précédentes, comprenant en outre un moyen de détermination d'un signal électrique amené par une déformation dans l'élément flexible (110) en se basant au moins en partie sur un mouvement de l'élément mobile (130).

13. Capteur de pression (100) selon la revendication précédente, comprenant en outre un moyen de transmission sans fil du signal électrique déterminé ou de n'importe quelle information en se basant sur le signal électrique déterminé en tant que signal analogique ou numérique.
